# EUROPEAN PATENT APPLICATION

(11) **EP 3 705 884 A1**
(43) Date of publication of application: **09.09.2020**
(21) Application number: 18886850.9
(22) Date of filing: 02.08.2018
(51) Int. Cl.: G01N 33/00, B01L 1/00

(54) **SIMPLE CHAMBER FOR FORMALDEHYDE OR VOC RELEASE TEST AND PRETREATMENT**

(30) Priority: 08.12.2017 CN 201711290125
(71) Applicant: Dongguan City Simplewell Technology Co., Ltd, Dongguan, Guangdong 523000 (CN)
(72) Inventor: XIA, Keyu, Dongguan Guangdong 523000 (CN)
(74) Representative: Lapienis, Juozas
(86) International application number: PCT/CN2018/098216
(87) International publication number: WO 2019/109648

(57) **Abstract**

A simple chamber for a formaldehyde or VOC release test and pretreatment, comprising a chamber (1) provided with a chamber door (2), an air inlet (3), and an exhaust port (4). At least one semiconductor refrigeration sheet (5) capable of cooling and/or heating or a liquid temperature regulating jacket (16) is mounted on the outer wall of the chamber (1), and a stirring fan (6) is mounted inside the chamber (1). A heat conduction zone in the semiconductor refrigeration sheet (5) or a temperature sensor (7) for temperature measurement of the liquid temperature regulating jacket (16) is mounted on the outer wall of the chamber (1). A temperature and humidity sensor (17) is installed inside the chamber (1), and a filtering duct (8) communicating with the interior of the chamber (1) is connected to the exterior of the chamber (1). An intake switch valve (9), an outlet switch valve (10), a filter device, and a filtering fan (11) are mounted on the filtering duct (8). The present invention has a simple structure and low costs, and can provide a constant temperature and/or constant humidity clean chamber (1) with a controllable temperature, relative humidity, and background concentration, thereby providing a stable standard test environment for testing, and avoiding the impact of environmental factors or other uncertainties on test results.

## Description

### TECHNICAL FIELD

The present invention relates to the technical field of test equipment, and particularly to a simple chamber for a formaldehyde or VOC release test and pretreatment.

### BACKGROUND

There are volatile harmful gases such as formaldehyde and volatile organic compounds (VOC) in the existing furniture, car accessories and other materials, and volatile harmful gases can cause damage on a human body if their concentrations exceed a certain range. Reasonable collection of volatile harmful gases such as formaldehyde and VOC is one of important links of a detection technique.

At present, there are many equipment for testing the release of volatile harmful gases such as formaldehyde and VOC in industries. However, the existing test equipment has the following defects: 1, structure is complicated, and cost is high; 2, environment factors such as temperature, humidity and air components of surrounding environment are prone to impacting the accuracy of the test; 3, when in testing, the test chamber does not discharge the original polluted air in the test chamber in advance, thereby impacting the accuracy of the test.

### SUMMARY

The object of the present invention is to overcome the above defects in the prior art, and provide a simple chamber for a formaldehyde or VOC release test and pretreatment, which is simple in structure and high in test accuracy.

In order to achieve the above object, the present invention provides a simple chamber for a formaldehyde or VOC release test and pretreatment, comprising a chamber provided with a chamber door, an air inlet used for introducing clean air having preset humidity and flow and an exhaust port used for exhausting and depressurization, wherein at least one semiconductor refrigeration sheet capable of cooling and/or heating or a liquid temperature regulating jacket is mounted on the outer wall of the chamber, and a stirring fan is mounted inside the chamber, a heat conduction zone in the semiconductor refrigeration sheet or a temperature sensor for temperature measurement of the liquid temperature regulating jacket is mounted on the outer wall of the chamber, a temperature and humidity sensor used for detecting temperature and humidity in the chamber is installed inside the chamber, a filtering duct communicating with the interior of the chamber is connected to the exterior of the chamber, and an intake switch valve, an outlet switch valve, a filter device located between the inlet switch valve and the outlet switch valve and a filtering fan are mounted on the filtering duct.

Preferably, the filter device comprises a formaldehyde and/or VOC filter and/or a dust filter which is connected to the filtering duct.

Preferably, the liquid temperature regulating jacket is a cavity where liquid circulates on at least one position of the outer wall of the chamber, a heating and cooling device used for controlling the temperature of the liquid is mounted inside the chamber and/or on a flow pipeline, and the temperature sensor senses the temperature of the liquid, controls the heating and cooling device to regulate the temperature of liquid flowing through the cavity, so as to indirectly control the temperature inside the chamber.

Preferably, the stirring fan is arranged as a frequency-conversion adjustable fan.

Preferably, a heater used for regulating the temperature in the chamber is mounted inside the chamber.

Preferably, an exhaust pump and/or exhaust valve used for controlling the pressure inside the chamber through exhausting is connected to the exhaust port of the chamber.

Preferably, a pressure sensor is mounted inside the chamber.

Preferably, an anti-adhesion coating used for preventing formaldehyde or VOC from adhering to the inner wall is provided on the inner wall of the chamber.

Preferably, the chamber is located in a constant temperature chamber, or an insulation layer is provided outside the chamber.

Preferably, the chamber consists of glass, a fluoride board and/or a stainless steel material.

Compared with the prior art, the present invention has the beneficial effects:
The present invention can avoid environment factors or other uncertainties to disturb test results, the semiconductor refrigeration sheet or the liquid temperature regulating jacket is capable of cooling and/or heating, the cooling or heating of the semiconductor refrigeration sheet or the liquid temperature regulating jacket can be controlled by detecting the temperature in the chamber so that the temperature in the chamber is constant, and the exterior filter device can filter the original polluted air in the chamber. The present invention has simple structure and low cost, can provide a constant temperature and/or constant humidity clean chamber having controllable temperature, relative humidity and background concentration, provides a stable standard test environment for testing, and avoids the impact of environment factors or other uncertainties on test results.

### BRIEF DESCRIPTION OF THE DRAWINGS

In order to more clearly illustrating the embodiments of the present invention or technical solution in the prior art, drawings needing to be used in embodiments or the prior art will be simply described below, obviously, drawings in the following description are some embodiments, persons of ordinary skill in the art can also obtain other drawings according to these drawings without creative efforts.
Fig.1 is a structure diagram of a simple chamber for a formaldehyde or VOC release test and pretreatment according to embodiment 1 of embodiments of the present invention;
Fig.2 is a structure diagram of a simple chamber for a formaldehyde or VOC release test and pretreatment according to embodiment 2 of embodiments of the present invention;

### DESCRIPTION OF THE EMBODIMENTS

To make the purpose, the technical solution and the advantages of the present invention more clear, a clear and complete description will be provided to the technical solution in the embodiments of the present invention below. Obviously, the described embodiments are some embodiments of the present invention rather than all the embodiments. Based on the embodiments in the present invention, other embodiments obtained by persons of ordinary skill in the art without creative efforts are all included in the protective scope of the present invention.

### Embodiment 1

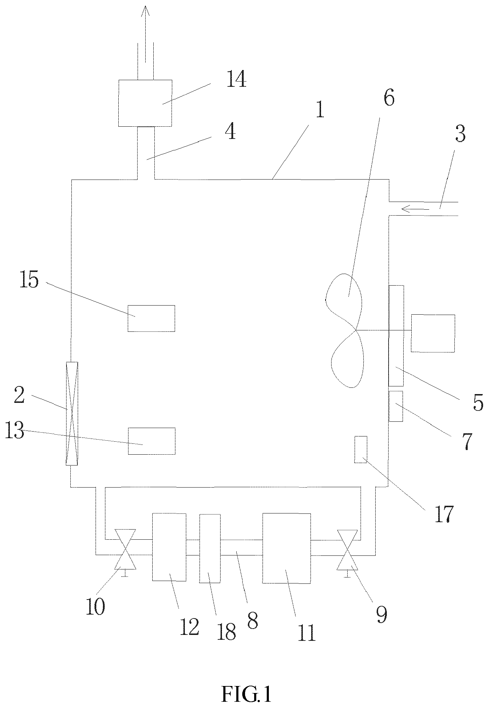

Referring to Fig.1, embodiment 1 of the present invention provides a simple chamber for a formaldehyde or VOC release test and pretreatment, comprising a chamber 1 provided with a chamber door 2, an air inlet 3 used for introducing clean air having preset humidity and flow and an exhaust port 4 used for exhausting and depressurization, wherein at least one semiconductor refrigeration sheet 5 capable of cooling and/or heating is mounted on the outer wall of the chamber 1, and a stirring fan 6 is mounted inside the chamber 1, a temperature sensor 7 located in a heat conduction zone of the semiconductor refrigeration sheet 5 is mounted on the outer wall of the chamber 1, and a temperature and humidity sensor 17 used for detecting temperature and humidity in the chamber is installed inside the chamber 1.

In this embodiment, the chamber 1 can consist of glass, a fluoride board and/or a stainless steel material. Preferably, a plurality of semiconductor refrigeration sheets 5 can be arranged. When one semiconductor refrigeration sheet 5 is malfunctioned, other semiconductor refrigeration sheets can still normally work. Of course, the quantity of the semiconductor refrigeration sheets 5 can be changed according to actual demands.

When in work, the semiconductor refrigeration sheets 5 conduct heat via the wall of the chamber, and the temperature sensor 7 can detect the temperature of the wall of the chamber, thereby controlling the cooling or heating of the semiconductor refrigeration sheets 5 so that the temperature in the chamber is constant, and also controlling the lowest temperature limit value of the wall of the chamber to prevent dew formation. The stirring fan 6 brings away the cooling capacity or heating capacity of the wall of the chamber via airflow, where, the stirring fan 6 can be preferably arranged as a frequency-conversion adjustable fan.

As shown in Fig.1, a filtering duct 8 communicating with the interior of the chamber 1 is connected to the exterior of the chamber 1, an intake switch valve 9, an outlet switch valve 10, a filter device located between the inlet switch valve 9 and the outlet switch valve 10 and a filtering fan 11 are mounted on the filtering duct 8. In this embodiment, especially, the filter device can comprise a formaldehyde and/or VOC filter 18 and/or a dust filter 12 which are connected to the filtering duct 8. The filtering fan 11 can quicken the circulation of air and improve the speed of filtration. Prior to testing, the exterior filter device can filter the original polluted air in the chamber, so as to improve the accuracy of test.

Besides the temperature in the chamber is controlled using the semiconductor refrigeration sheets 5, a heater 13 used for regulating the temperature in the chamber is mounted inside the chamber 1.

As shown in Fig.1, an exhaust pump 14 and/or an exhaust valve is connected to the exhaust port 4 of the chamber 1. Correspondingly, a pressure sensor 15 is mounted in the chamber 1. When in work, exhausting via the exhausting pump 14 or regulating the exhaust valve can control the pressure inside the chamber 1 and discharge the original polluted air in the chamber. Prior to testing, clean air enters the chamber via the air inlet 3 to discharge the original polluted air in the chamber.

Preferably, an anti-adhesion coating used for preventing formaldehyde or VOC from adhering to the inner wall is provided on the inner wall of the chamber 1 to further improve the accuracy of test.

When in actual use, the chamber 1 can be placed in the temperature constant chamber, or an insulation layer is provided outside the chamber 1 for thermal insulation.

### Embodiment 2

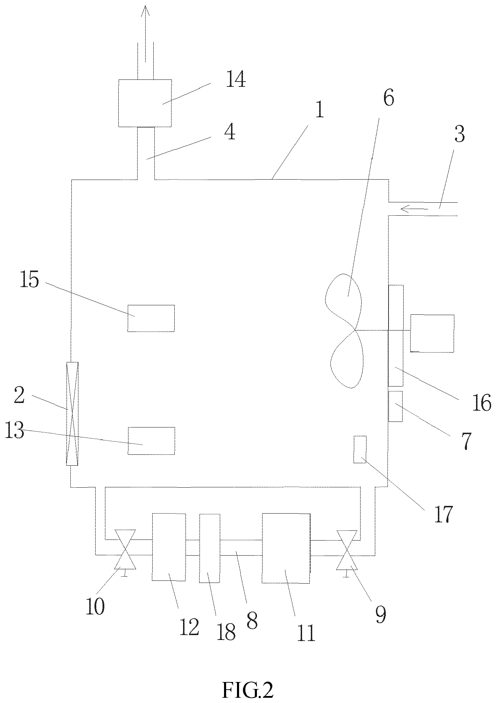

Referring to Fig.2, embodiment 2 of the present invention provides an another simple chamber for a formaldehyde or VOC release test and pretreatment, comprising a chamber 1 provided with a chamber door 2, an air inlet 3 used for introducing clean air having preset humidity and flow and an exhaust port 4 used for exhausting and depressurization, wherein at least one liquid temperature regulating jacket 16 capable of cooling and/or heating is mounted on the outer wall of the chamber 1, and a stirring fan 6 is mounted inside the chamber 1, a temperature sensor 7 used for temperature measurement of the liquid temperature regulating jacket 16 is mounted on the outer wall of the chamber 1, and a temperature and humidity sensor 17 used for detecting temperature and humidity in the chamber is installed inside the chamber 1.

In this embodiment, the liquid temperature regulating jacket 16 is a cavity where liquid circulates on at least one position of the outer wall of the chamber, a heating and cooling device used for controlling the temperature of the liquid is mounted inside the chamber, the temperature sensor 7 senses the temperature of the liquid, and controls the heating and cooling device to regulate the temperature of liquid flowing through the cavity, so as to indirectly control the temperature inside the chamber.

Herein, it is noted that other structures of the simple chamber for a formaldehyde or VOC release test and pretreatment in this embodiment 2 are the same as those of the simple chamber for a formaldehyde or VOC release test and pretreatment in the above embodiment 1, and are not repeatedly described.

In summary, the present invention has a simple structure and low costs, and can provide a constant temperature and/or constant humidity clean chamber 1 with a controllable temperature, relative humidity and background concentration, thereby providing a stable standard test environment for testing, and avoiding the impact of environmental factors or other uncertainties on test results.

The above embodiments are preferred embodiments of the present invention, however, the embodiments of the present invention are not limited by the above embodiments, and any other variations, modifications, substitutions, combinations and simplifications made without departing from the spirit and principle of the present invention are all equivalent replacement manners, and included within the protective scope of the present invention.

## Claims

1. A simple chamber for a formaldehyde or VOC release test and pretreatment, comprising: a chamber (1) provided with a chamber door (2), an air inlet (3) used for introducing clean air having preset humidity and flow and an exhaust port (4) used for exhausting and depressurization, wherein at least one semiconductor refrigeration sheet (5) capable of cooling and/or heating or a liquid temperature regulating jacket (16) is mounted on the outer wall of the chamber (1), and a stirring fan (6) is mounted inside the chamber (1), a heat conduction zone in the semiconductor refrigeration sheet (5) or a temperature sensor (7) for temperature measurement of the liquid temperature regulating jacket (16) is mounted on the outer wall of the chamber (1), and a temperature and humidity sensor (17) used for detecting temperature and humidity in the chamber is installed inside the chamber (1).

2. The simple chamber for a formaldehyde or VOC release test and pretreatment according to claim 1, wherein a filtering duct (8) communicating with the interior of the chamber (1) is connected to the exterior of the chamber (1), an intake switch valve (9), an outlet switch valve (10), a filter device located between the inlet switch valve (9) and the outlet switch valve (10) and a filtering fan (11) are mounted on the filtering duct (8), and the filter device comprises a formaldehyde and/or VOC filter (18) and/or dust filter (12) which are connected to the filtering duct (8).

3. The simple chamber for a formaldehyde or VOC release test and pretreatment according to claim 1, wherein the liquid temperature regulating jacket (16) is a cavity where liquid circulates on at least one position of the outer wall of the chamber, a heating and cooling device used for controlling the temperature of the liquid is mounted inside the chamber and/or on a flow pipeline, the temperature sensor (7) senses the temperature of the liquid, controls the heating and cooling device to regulate the temperature of liquid flowing through the cavity, so as to indirectly control the temperature inside the chamber.

4. The simple chamber for a formaldehyde or VOC release test and pretreatment according to claim 1, wherein the stirring fan (6) is arranged as a frequency-conversion adjustable fan.

5. The simple chamber for a formaldehyde or VOC release test and pretreatment according to claim 1, wherein a heater (13) used for regulating the temperature in the chamber is mounted inside the chamber (1).

6. The simple chamber for a formaldehyde or VOC release test and pretreatment according to claim 1, wherein an exhaust pump (14) and/or exhaust valve used for controlling the pressure inside the chamber (1) through exhausting is connected to the exhaust port (4) of the chamber (1).

7. The simple chamber for a formaldehyde or VOC release test and pretreatment according to claim 6, wherein a pressure sensor (15) is mounted inside the chamber (1).

8. The simple chamber for a formaldehyde or VOC release test and pretreatment according to claim 1, wherein an anti-adhesion coating used for preventing formaldehyde or VOC from adhering to the inner wall is provided on the inner wall of the chamber (1).

9. The simple chamber for a formaldehyde or VOC release test and pretreatment according to claim 1, wherein the chamber (1) is located in a constant temperature chamber, or an insulation layer is provided outside the chamber (1).

10. The simple chamber for a formaldehyde or VOC release test and pretreatment according to claim 1, wherein the chamber (1) consists of glass, a non-metal board (film) and/or a stainless steel material.
